(19) Europäisches Patentamt
European Patent Office
Office européen des brevets

(11) Veröffentlichungsnummer : **0 410 214 B1**

# (12) EUROPÄISCHE PATENTSCHRIFT

(45) Veröffentlichungstag der Patentschrift :
**18.08.93 Patentblatt 93/33**

(51) Int. Cl.$^5$ : **C07C 271/08**

(21) Anmeldenummer : **90113304.1**

(22) Anmeldetag : **12.07.90**

## (54) Verfahren zur Herstellung N,N-disubstituierter Mono- und Oligourethane.

(30) Priorität : **25.07.89 DE 3924545**

(43) Veröffentlichungstag der Anmeldung :
**30.01.91 Patentblatt 91/05**

(45) Bekanntmachung des Hinweises auf die Patenterteilung :
**18.08.93 Patentblatt 93/33**

(84) Benannte Vertragsstaaten :
**AT BE CH DE ES FR GB IT LI NL SE**

(56) Entgegenhaltungen :
**EP-A- 0 298 636**
**DE-A- 3 609 813**

(56) Entgegenhaltungen :
**US-A- 4 268 684**
**US-A- 4 567 287**
**CHEMICAL ABSTRACTS, Band 92, Nr. 7, 18. Februar 1980, Columbus, Ohio, USA S. JULIA et al. "Phase-trans- fer catalysis IV. N-alkylationof naphthyl carbamates" Seite 644, Spalte 2, Zusammenfassung-Nr. 58 478c**

(73) Patentinhaber : **BAYER AG**
**D-51368 Leverkusen (DE)**

(72) Erfinder : **Reiff, Helmut, Dr.**
**Paul-Klee-Strasse 68i**
**D-5090 Leverkusen 1 (DE)**
Erfinder : **Dieterich, Dieter, Prof. Dr.**
**Henry-Theodor-von-Boettinger-Strasse 16**
**D-5090 Leverkusen (DE)**



Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung N,N-disubstituierter Mono- und Oligourethane durch Umsetzung N-aromatisch, -aliphatisch, cycloaliphatisch sowie -araliphatisch substituierter Mono- und Oligourthane mit Dialkylcarbonaten in Gegenwart mindestens äquivalenter Mengen an festem Alkali- bzw. Erdalkalicarbonat, insbesondere Kalium- und/oder Natriumcarbonat in überschüssigem Dialkylcarbonat und/oder in einem geeigneten Lösungsmittel und in Gegenwart eines Phasentransferkatalysators.

Es ist bereits bekannt, daß Monourethane mit niederen Alkylhalogeniden bzw. Alkylsulfaten zu N,N-disubstituierten Monourethanen umgesetzt werden können (vgl. U. Petersen in Houben-Weyl, Bd. E 4, S. 169 Herausgeber Hagemann). Nachteilig bei den bekannten Verfahren ist jedoch, daß gute Ergebnisse nur dann erzielt werden, wenn dazu spezielle, relaiv teure Basen wie Metallhydride, z.B. NaH, eingesetzt werden. Wegen der unter diesen Reaktionsbedingungen dominierenden Nebenreaktion der Olefinbildung beim Einsatz sekundärer Alkylierungsmittel sind diese Verfahren darüber hinaus auf den Einsatz primärer Alkylierungsmittel beschränkt.

Es ist weiterhin bekannt, daß N-arylsubstituierte Monourethane mit Alkylhalogeniden bzw. Dialkylsulfaten unter den Bedingungen der Phasentransferkatalyse N-alkyliert werden können. Die Methode versagt jedoch bei N-aliphatisch substituierten Urethanen völlig (Vgl. S. Julia, A. Ginebreda, Anale de Quimica (Madrid), Vol. 75, S. 348, Zeile 7-13).

Ferner wird in der DE-OS 36 09 813 ein Verfahren zur Herstellung N,N-disubstituierter Mono- und Oligourethane beschrieben, das dadurch gekennzeichnet ist, daß N-aliphatisch, -cycloaliphatisch sowie araliphatisch substituierte Mono- und Oligourethane mit Alkylierungsmitteln in Gegenwart mindestens äquivalenter Mengen an festem Metallhydroxid in Substanz oder in einem aprotischen organischen Lösungsmittel, gegebenenfalls in Gegenwart eines Phasentransferkatalysators umgesetzt werden.

Allen bekannten Verfahren ist gemeinsam, daß die Alkylierungen letztlich mit Alkylierungsmitteln durchgeführt werden. Diese sind bekanntlich fast alle physiologisch bedenklich. N-Alkylierungen werden beispielsweise mit Dimethylsulfat oder Methylchlorid bzw. Methyljodid durchgeführt.

Aufgabe der vorliegenden Erfindung war es, ein wirtschaftliches Verfahren zur Alkylierung N-aromatisch bzw, N-aliphatisch substituierter Urethane bereitzustellen, das die beschriebenen Nachteile vermeidet, Es wurde nun überraschenderweise gefunden, daß man die gewünschten N,N-disubstituierten Mono- und Oligourethane erhält, wenn N-aromatisch, -aliphatisch, -cycloaliphatisch sowie -araliphatisch substituierte Mono- und Oligourethane mit Dialkylcarbonaten in Gegenwart mindestens äquivalenter Mengen an festem Alkali- bzw, Erdalkalicarbonat, insbesondere Kalium- und/oder Natriumcarbonat in überschüssigem Dialkylcarbonat und/oder in einem aprotischen organischen Lösungsmittel und in Gegenwart eines Phasentransferkatalysators umgesetzt werden.

Gegenstand der Erfindung ist also ein Verfahren zur Herstellung N,N-di-substituierter Mono- und Oligourethane der allgemeinen Formeln

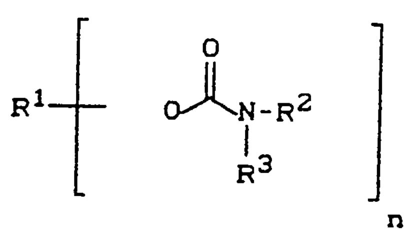

und

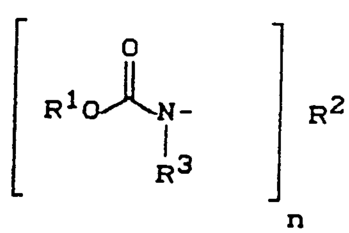

mit n = 1, 2, 3, 4, 5, 6

$R^1$ der nach Subtraktion der OH-Gruppe verbleibende Rest eines ein- bis sechswertigen aromatischen, aliphatischen, cycloaliphatischen oder araliphatischen Alkohols,

$R^2$ der nach Subtraktion der NCO-Gruppen verbleibende Rest eines aromatischen, aliphatischen, cycloaliphatischen oder araliphatischen Mono- oder Polyisocyanats,

$R^3$ einen gegebenenfalls ungesättigten, gegebenenfalls verzweigten aliphatischen oder araliphatischen Kohlenwasserstoffrest mit 1-18, bevorzugt 1-10 C-Atomen, der weitere funktionelle Gruppen tragen kann,

bedeutet,

welches dadurch gekennzeichnet ist, daß man die Urethane mit Dialkylcarbonaten in Gegenwart mindestens äquivalenter Mengen eines festen Alkali- bzw, Erdalkalicarbonats in Substanz oder in überschüssigem Dialkylcarbonat und/oder in einem aprotischen organischen Lösungsmittel, in Gegenwart eines Phasentransferkatalysators, umsetzt.

Zur Erzielung guter Ausbeuten ist es besonders vorteilhaft, das erfindungsgemäße Verfahren derart durchzuführen, daß man das Ausgangsurethan, das Dialkylcarbonat und den Phasentransferkatalysator in einem aprotischen organischen Lösungsmittel - bevorzugt werden Dimethylsulfoxid, Chlorbenzol, Dimethylformamid und N-Methylpyrrolidon - oder in überschüssigem Dialkylcarbonat löst und dann das trockene, pulverförmige Alkali- bzw, Erdalkalicarbonat, bevorzugt Kaliumcarbonat und/oder Natriumcarbonat zugibt und anschließend bis zum vollständigen Umsatz unter Erwärmen auf 90 - 140°C nachrührt,

Im Vergleich zu den bekannten Verfahren liefert das erfindungsgemäße Verfahren überraschend und auf einfache Weise N,N-disubstituierte Urethane in sehr guten Ausbeuten, und es kommen im Gegensatz zu den bekannten Verfahren statt gefährlicher Metallhydride und Alkylierungsmittel preisgünstige, ungefährlichere und einfacher handhabbare Substanzen wie Alkali- bzw, Erdalkalicarbonate und Dimethylcarbonat zum Einsatz.

Es muß ganz besonders überraschen, daß nach dem erfindungsgemäßen Verfahren im Gegensatz zur Literatur auch N-aliphatischen substituierte Urethane alkyliert werden können, wenn auch längere Reaktionszeiten in Kauf genommen werden müssen.

Als Ausgangsmaterial für das erfindungsgemäße Verfahren kommen Urethane in Betracht, die sich z.B. durch Umsetzung von aromatischen, araliphatischen oder aliphatischen Mono- und Oligoisocyanaten mit ein- bis sechswertigen Alkoholen nach bekannten Verfahren in der Schmelze oder in Lösung, gegebenenfalls in Gegenwart eines Katalysators, herstellen lassen.

Diese Urethane können aber beispielsweise auch durch Kondensation primärer Mono- oder Oligoamine mit Chlorameisensäureestern ein- oder zwei- bis sechswertiger Alkohole hergestellt werden. Selbstverständlich kann man zu ihrer Herstellung auch Carbamidsäurechloride mit Alkoholen umsetzen.

Zur Herstellung der Ausgangsurethane für das erfindungsgemäße Verfahren können beispielsweise eingesetzt werden:
Alkohole der Formel

$$R^1(OH)_n$$

in der
n = ganze Zahlen von 1 bis 6, vorzugsweise 1-4 und
$R^1$ einen aromatischen Kohlenwasserstoffrest mit 6-18, bevorzugt 6-13 C-Atomen, einen aliphatischen Kohlenwasserstoffrest mit 1-18, vorzugsweise 1-6 C-Atomen, einen cycloaliphatischen Kohlenwasserstoffrest mit 4-30, vorzugsweise 6-15 C-Atomen oder einen araliphatischen Kohlenwasserstoffrest mit 7-30, bevorzugt 7-15 C-Atomen bedeutet.

Hier kommen beispielsweise einwertige Alkohole in Frage, wie sie in Ullmanns Enzyklopädie der Technischen Chemie, Bd. 7, S. 205-206, 4. Auflage, 1974 beschrieben sind, außerdem Phenole sowie substituierte Phenole.

Als mehrwertige Alkohole kommen z.B. Ethylenglykol, Propylenglykol-(1,2) und -(1,3), Butylenglykol-(1,4) und -(2,3), Hexandiol-(1,6), Octandiol-(1,8), Neopentylglykol, 1,4-Bis-hydroxymethylcyclohexan, 2-Methyl-1,3-propandiol, Glycerin, Trimethylolpropan, Hexantriol-(1,2,6), Butantriol-(1,2,4), Trimethylolethan, Pentaerythrit, Chinit, Mannit und Sorbit, Formit, Methylglykosid und/oder 1,4-, 3,6-Dianhydrohexite, außerdem mehrwertige Phenole wie z.B. Brenzcatechin, Resorcin, Hydrochinon sowie mehrkernige Phenole z.B. vom Typ des Bisphenol A in Frage.

Selbstverständlich können auch Mischungen dieser Alkohole eingesetzt werden.

Isocyanate zur Herstellung der Ausgangsprodukte haben die allgemeine Formel $R^2(NCO)_n$, in der
n = ganze Zahlen von 1.6, vorzugsweise 1-3 und
$R^2$ einen aromatischen, araliphatischen, cycloaliphatischen oder aliphatischen Kohlenwasserstoffrest mit 1-28, vorzugsweise 2-18 C-Atomen bedeutet.

Beispielsweise können eingesetzt werden: Isocyanatomethan, -ethan, -propan, ,butan, -pentan, -hexan, 6-Chlorhexylisocyanat, Isocyanatocyclohexan, Benzylisocyanat, Tetramethylendiisocyanat; Hexamethylendiisocyanat, Dekamethylendiisocyanat; 1,3-Di-(3-isocyanatapropoxy)-2,2-dimethylpropan; Cyclohexandiisocyanat-(1,4); Methylcyclohexandiisocyanat-(2,4); Methylcyclohexandiisocyanat-(2,6); 1,3-Diisocyanatocyclohexan, Gemische aus Methylcyclohexandiisocyanat-(2,4) und Methylcyclohexandiisocyanat-(2,6); Dicyclohexylmethan-4,4'-diisocyanat; 1-Isocyanato-3-isocyanatomethyl-3,5,5-trimethylcyclohexan (Isophorondiisocyanat); 1,2-Di-(isocyanatomethyl)-cyclobutan; m- und p-Xylylendiisocyanat sowie $\alpha,\alpha,\alpha',\alpha'$-Tetramethyl-m- und/oder -p-xylylendiisocyanat oder -hexahydroxylylendiisocyanat.

Weiter können bevorzugt verwendet werden Phenylisocyanat, die isomeren Tolylisocyanate, 3-Chlorphenylisocyanat, 4-Chlorphenylisocyanat, 3,4-Dichlorphenylisocyanat, die isomeren Nitrophenylisocyanate, die isomeren Naphthylisocyanate usw.

Als einzusetzende Polyisocyanat-Ausgangskomponenten kommen aliphatische, cycloaliphatische,

araliphatische, aromatische und heterocyclische Polyisocyanate in Betracht, wie sie z.B. von W. Siefken in Justus Liebigs Annalen der Chemie, 562, Seiten 75 bis 136, beschrieben werden, beispielsweise Äthylen-diisocyanat, 1,4-Tetramethylendiisocyanat, 1,6-Hexamethylendiisocyanat, 1,12-Dodecandiisocyanat, Cyclobutan-1,3-diisocyanat, Cyclohexan-1,3- und -1,4-diisocyanat sowie beliebige Gemische dieser Isomeren, 1-Isocyanato-3,3,5-trimethyl-5-isocyanatomethyl-cyclohexan (DAS 202 785, amerikanische Patentschrift 3 401 190), 2,4- und 2,6-Hexahydrotoluylendiisocyanat sowie beliebige Gemische dieser Isomeren, Hexahydro-1,3- und-/oder 4,4-phenylen-diisocyanat, Perhydro-2,4'- und/oder -4,4'-diphenylmethan-diisocyanat, 1,3- und 1,4-Phenylendiisocyanat, 2,4- und 2,6-Toluylendiisocyanat sowie beliebige Gemische dieser Isomeren, Diphenylmethan-2,4'- und/oder -4,4'-diisocyanat, Naphthylen-1,5-diisocyanat, Triphenylmethan-4,4',4"-triisocyanat, Polyphenyl-polymethylen-polyisocyanate, wie sie durch Anilin-Formaldehyd-Kondensation und anschließende Phosgenierung erhalten und z.B. in den britischen Patentschriften 874 430 und 848 671 beschrieben werden, m- und p-Isocyanatophenylsulfonyl-isocyanate gemäß der amerikanischen Patentschrift 3 454 606, perchlorierte Arylpolyisocyanate, wie sie z.B. in der deutschen Auslegeschrift 1 157 601 (amerikanische Patentschrift 3 277 138) beschrieben werden, Carbodiimidgruppen aufweisende Polyisocyanate, wie sie in der deutschen Patentschrift 1 092 007 (amerikanische Patentschrift 3 152 162) beschrieben werden, Diisocyanate, wie sie in der amerikanischen Patentschrift 3 492 330 beschrieben werden, Allophanatgruppen aufweisende Polyisocyanate, wie sie z.B. in der britischen Patentschrift 994 890, der belgischen Patentschrift 761 626 und der veröffentlichten holländischen Patentanmeldung 7 102 524 beschrieben werden, Isocyanuratgruppen aufweisende Polyisocyanate, wie sie z.B. in der amerikanischen Patentschrift 3 001 973, in den deutschen Patentschriften 1 022 789, 1 222 067 und 1 027 394 sowie in den deutschen Offenlegungsschriften 1 929 034 und 2 004 048 beschrieben werden, Urethangruppen aufweisende Polyisocyanate, wie sie z.B. in der belgischen Patentschrift 752 261 oder in der amerikanischen Patentschrift 3 394 164 beschrieben werden, acylierte Harnstoffgruppen aufweisende Polyisocyanate gemäß der deutschen Patentschrift 1 230 778, Biuretgruppen aufweisende Polyisocyanate, wie sie z.B. in der deutschen Patentschrift 1 101 394 (amerikanische Patentschriften 3 124 605 und 3 201 372) sowie in der britischen Patentschrift 889 050 beschrieben werden, durch Telomerisationsreaktion hergestellte Polyisocyanate, wie sie z.B. in der amerikanischen Patentschrift 3 654 106 beschrieben werden, Estergruppen aufweisende Polyisocyanate, wie sie z.B. in den britischen Patentschriften 965 474 und 1 072 956, in der amerikanischen Patentschrift 3 567 763 und in der deutschen Patentschrift 1 231 688 genannt werden, Umsetzungsprodukte der obengenannen Isocyanate mit Acetalen gemäß der deutschen Patentschrift 1 072 385, polymere Fettsäureester enthaltende Polyisocyanate gemäß der amerikanischen Patentschrift 3 455 883.

Es ist auch möglich, die bei der technischen Isocyanatherstellung anfallenden Isocyanatgruppen aufweisenden Destillationsrückstände, gegebenenfalls gelöst in einem oder mehreren der vorgenannten Polyisocyanate, einzusetzen. Ferner ist es möglich, beliebige Mischungen der vorgenannten Polyisocyanate zu verwenden.

Besonders bevorzugt werden in der Regel die technisch leicht zugänglichen Polyisocyanate, z.B. das 2,4- und 2,6-Toluylendiisocyanat sowie beliebige Gemische dieser Isomeren ("TDI"), Polyphenyl-polymethylen-polyisocyanate, wie sie durch Anilin-Formaldehyd-Kondensation und anschließende Phosgenierung hergestellt werden ("rohes MDI") und Carbodiimidgruppen, Urethangruppen, Allophanatgruppen, Isocyanatgruppen, Harnstoffgruppen oder Biuretgruppen aufweisenden Polyisocyanate ("modifizierte Polyisocyanate").

Bei cycloaliphatischen Diisocyanaten können beliebige Stereoisomere oder ihre Gemische Verwendung finden.

Selbstverständlich können auch Mischungen dieser Isocyanate eingesetzt werden.

Es kommen erfindungsgemäß weiterhin Dialkylcarbonat der Formel

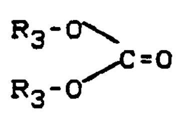

zum Einsatz,

in der

$R^3$ einen gegebenenfalls ungesättigten, gegebenenfalls verzweigten aliphatischen oder araliphatischen Kohlenwasserstoffrest mit 1-18, bevorzugt 1-10 C-Atomen bedeutet.

Selbstverständlich darf der Kohlenwasserstoff $R^3$ auch weitere funktionelle Gruppen tragen, wenn diese unter den erfindungsgemäßen Reaktionsbedingungen inert sind oder in definierter Weise mit den erfindungsgemäßen Reagenzien reagieren, z.B. Nitro-, bestimmte Ester-, Urethan-Amidgruppen und Sulfonylgruppen, nicht aktiviertes, aromatisch gebundenes Halogen, Epoxidgruppen, Aziridingruppen, Ethergruppen, Thioethergruppen und dergleichen mehr.

Als Dialkylcarbonate kommen beispielsweise in Frage: Dimethylcarbonat, Diethylcarbonat, Di-n-propylcarbonat, Dibutylcarbonat, Dipentylcarbonat usw. Geeignet sind auch Diallylcarbonat, Divinylcarbonat, gegebenenfalls substituierte Dibenzylcarbonate, Bis-Nitrophenylcarbonate usw.

Selbstverständlich können auch Mischungen dieser Carbonate eingesetzt werden.

Ganz besonders bevorzugte Dialkylcarbonate sind Dimethylcarbonat und Diethylcarbonat.

Das erfindungsgemäße Verfahren kann entweder in einem aprotischen organischen Lösungsmittel oder in überschüssigem, flüssigem Dialkylcarbonat in Gegenwart eines Phasentransferkatalysators ausgeübt werden.

Erfindungsgemäß werden als Basen feste, fein pulverisierte Alkali- bzw. Erdalkalimetallcarbonate wie z.B. Kalium- und Natriumcarbonat eingesetzt. Kaliumcarbonat wird bevorzugt. Selbstverständlich kann man z.B. auch gegebenenfalls anteilig Lithium-, Calcium-, Magnesium- und Bariumcarbonat einsetzen. Es kann in manchen Fällen vorteilhaft sein, Mischungen dieser Carbonate einzusetzen.

Erfindungsgemäß werden weiterhin Phasentransferkatalysatoren eingesetzt.

Solche Katalysatoren werden z.B. in E.V. und S.S. Dehmlow, Phase Transfer Catalysis, 2. Auflage, Verlag Chemie 1983, beschrieben. Geeignete Katalysatoren sind quaternäre Ammonium- oder Phosphoniumsalze der Formel

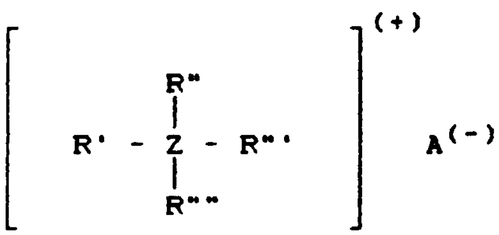

in welcher

Z für Stickstoff oder Phosphor steht,

R', R", R"' und R"" für gleiche oder verschiedene Reste stehen und Alkylgruppen mit 1 bis 18 Kohlenstoffatomen bedeuten, wobei einer der Reste auch für einen araliphatischen Rest mit 7 bis 15 Kohlenstoffatomen stehen kann, und wobei die Summe der Kohlenstoffatome der vier Reste vorzugsweise bei 12 bis 29 liegt und A ein einwertiges Anion, beispielsweise Fluorid, Chlorid, Bromid, Iodid oder Hydrogensulfat bedeutet.

Typische Beispiele geeigneter Katalysatoren sind N-Benzyl-N,N,N-triethyl-ammoniumchlorid oder -bromid, N-Benzyl-N-dodecyl-N,N-dimethyl-ammoniumchlorid oder -bromid, N,N,N,N-Tetra-n-hexyl-ammoniumchlorid oder -bromid, N-Benzyl-N,N,N-tri-n-octyl-ammoniumchlorid oder -bromid oder die diesen Ammoniumsalzen entsprechenden Phosphoniumsalze.

Weitere erfindungsgemäße Phasentransferkatalysatoren sind beispielsweise:

Tetrabutylammoniumbromid, Tetrabutylammoniumjodid, Tetrabutylammoniumbromid + DMF, Tetrabutylammoniumchlorid, Tetrabutylammoniumhydrogensulfat, Tetrabutylammoniumbromid/NaJ 1:1, Tetraethylammoniumchlorid, Zephirolsaccharinat, Tricaprolylmethylammoniumchlorid, Kronenether 18, Benzyltrimethylammoniumfluorid, Cetyltrimethylammoniumbromid, Tetrabutylphosphoniumbromid, Trimethylbenzylammoniumchlorid.

Bevorzugte Phasentransferkatalysatoren sind Tetrabutylammoniumchlorid, Tetrabutylammoniumbromid, Tetrabutylammoniumjodid, Triethylbenzylammoniumchlorid und -bromid sowie Trimethyl ammoniumchlorid und -bromid.

Die oben beschriebenen Ausgangsurethane können mit dem Dialkylcarbonat in (bezogen auf die Anzahl im Molekül vorhandener Urethangruppen stöchiometrischer Menge, im Überschuß oder Unterschuß umgesetzt werden. Bevorzugt wird ein Verhältnis von 0,9-15 Mol, ganz besonders bevorzugt 1 - 5 Mol Dialkylcarbonat pro Mol Urethangruppen.

Selbstverständlich wird bei Verwendung eines Unterschusses an Dialkylcarbonat nur eine Teilalkylierung erreicht werden, während die Verwendung eines größeren Überschusses wegen der Recyclisierbarkeit nicht unwirtschaftlich sein muß.

Die Umsetzung wird im allgemeinen bei einer Temperatur von 20 - 180°C, bevorzugt bei 80 - 140°C, drucklos, kontinuierlich oder diskontinuierlich durchgeführt.

Zweckmäßig führt man die Reaktion besonders bevorzugt in überschüssigem Dialkylcarbonat oder aber in einem polaren aprotischen, organischen Lösungsmittel durch.

Geeignet sind solche aprotischen organischen Lösungsmittel, die unter den erfindungsgemäßen Reaktionsbedingungen inert sind und z.B. in Ullmanns Enzyklöpädie der Technischen Chemie Bd. 14, Auflage, Verlag Chemie 1978, S. 305 beschrieben sind. Als Lösungsmittel kommen z.B. in Frage: Benzol, Toluol, Xylol, Ethylbenzyl, Cumol, Methylenchlorid, Chloroform, Dichlorbenzol, Trichlorbenzol, Nitrobenzol, Aceton, Methylethylketon, Diethylketon, Cyclohexanon, Diethylether, Diisopropylether, Tetrahydrofuran, Dioxan, Dimethylformamid, Dimethylacetamid, Dimethylsulfoxid, Tetramethylensulfon, Furfurol, Nitromethan, Nitroethan, Nitropropan, N-Methylpyrrolidon, Hexamethylenphosphonsäuretriamid.

Bevorzugt werden Dimethylsulfoxid, Chlorbenzol, Dimethylformamid, N-Methylpyrrolidon und Tetramethylensulfon.

Selbstverständlich können auch Mischungen dieser Lösungsmittel verwendet werden.

Die beispielhaft genannten quarternären Ammonium- oder Phosphoniumsalze werden bei der Durchführung des erfindungsgemäßen Verfahrens vorzugsweise in Substanz und vorzugsweise einer Menge von 1 bis 10 Mol-%, bezogen auf die Molzahl der vorhandenen Urethangruppen eingesetzt.

Zur Durchführung des erfindungsgemäßen Verfahrens kann man beispielsweise so vorgehen, daß man das Urethan, das Dialkylcarbonat und den Katalysator im gewählten Lösungsmittel vorlegt und das feste, trockene, möglichst feingemahlene Alkalicarbonat unter Rühren in einer Portion, portionsweise oder kontinuierlich zugibt. Anschließend rührt man bei erhöhter Temperatur, beispielsweise 80-140°C so lange nach, bis die dünnschichtchromatographische oder gaschromatographische Analyse vollständigen Umsatz anzeigt.

Die Aufarbeitung kann nach an sich bekannten Verfahren erfolgen. Bei Verwendung von mit Wasser mischbarem Lösungsmittel kann man im Fall fester, in Wasser unlöslicher Reaktionsprodukte das Reaktionsgemisch in Wasser einrühren und das ausgefallene Reaktionsprodukt in üblicher Weise durch Absaugen isolieren. Im Fall öliger Reaktionsprodukte ist dagegen eine extraktive Aufarbeitung nach üblichen Methoden zweckmäßig. Die Rohprodukte können falls erforderlich, in üblicher Weise, z.B. durch Umkristallisation oder Destillation, gereinigt werden.

Die nach dem Verfahren der Erfindung herstellbaren N,N-disubstituierten Urethane sind Wirkstoffe und wertvolle Ausgangsstoffe für die Herstellung von Farbstoffen, Pharmazeutika und thermostabilen Kunststoffen. Insbesondere zeigen die erfindungsgemäßen N,N-disubstituierten Urethane größere thermische, thermooxidative und photo-oxidative Stabilität (vgl. R. Vieweg, A. Höchtlen, Kunststoff Handbuch Bd. VII, Polyurethane, Hauser Verlag München 1966, S. 11, S. 21), sowie ein günstigeres Brandverhalten als die entsprechende N-monosubstituierten Urethane.

Durch Hydrolyse der N,N-disubstituierten Urethane können die entsprechend substituierten sekundären Amine hergestellt werden, die ebenfalls wichtige Ausgangsprodukte für die Synthese von Wirkstoffen sowie die Herstellung und Formulierung von Kunststoffen darstellen.

Alle Reaktionsprodukte wurden gaschromatographisch oder dünnschichtchromatographisch auf ihre Reinheit überprüft und ihre Identität durch Aufnahme von IR- und NMR-Spektrum gesichert.

Gerade die IR-Spektroskopie gestattet eine bequeme Umsatzkontrolle: Im Verlauf der Reaktion verschwinden die für N-monosubstituierte Urethane charakteristischen Banden bei 3200-3500 $cm^{-1}$ ($\nu$ N-H) und 1530-1560 $cm^{-1}$ ($\delta$ N-H).

Der Gegenstand der Erfindung soll anhand der folgenden Beispiele noch näher erläutert werden.

Beispiel 1

Herstellung von N-Methyl-Phenylmethylurethan.

15,1 g (0,1 mol) Phenylmethylurethan werden zusammen mit 100 ml Dimethylcarbonat vorgelegt. Bei Raumtemperatur setzt man unter Rühren nacheinander 5 g (0,036 mol) Kaliumcarbonat (gerieben und getrocknet) und 1 g (0,003 mol) Tetrabutylammoniumbromid zu. Nach Erwärmen auf Rückfluß (95 - 97°C) wird 7 Tage bei dieser Temperatur nachgerührt.

Die Lösung wird nun heiß filtriert, abgekühlt und am Rotationsverdampfer vom Lösungsmittel befreit. Man erhält 14,8 g eines hellbraunen, klaren Öles, welches am Kugelrohrofen (Fa. Büchi, Modell GKR-50) bei ca. 150°C (0,01 mbar) destilliert wird. Die Ausbeute beträgt 13 g (88% d. Th.) des Produktes der Formel

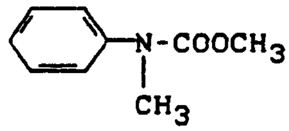

Beispiel 2

Man arbeitet völlig analog Beispiel 1, jedoch in Gegenwart von 100 ml N-Methylpyrrolidon (NMP). Nach zwei Tagen Rückfluß ist gaschromatographisch kein Ausgangsprodukt mehr nachweisbar. Die Ausbeute beträgt 99% d. Th. N-Methyl-Phenylmethylurethan.

Beispiel 3

Verwendet man 100 ml Dimethylsulfoxid (DMSO), so erhält man - sonst völlig analog Beispiel 1 - 96% d. Th.

Beispiel 2 und 3 zeigen eine deutliche Beschleunigung der Reaktion durch polare, aprotische Solventien.

Beispiel 4-14

Die Beispiele 4-14 werden tabellarisch zusammengefaßt. Bei allen Reaktionen wird völlig analog Beispiel 1 Dimethylcarbonat verwendet. Die Ausgangsurethane werden variiert, um die Vielfalt der Produkte aufzuzeigen. Unter Bemerkung werden gegebenenfalls Cosolvens, gegebenenfalls Nebenprodukte und/oder gegebenenfalls Ausgangsmaterial aufgeführt.

Die verwendeten Phasentransferkatalysatoren werden wie folgt abgekürzt:

TBAB Tetrabutylammoniumbromid
TMBAC Trimethylbenzylammoniumchlorid
TBAJ Tetrabutylammoniumjodid
CTMAB Cetyltrimethylammoniumbromid

Urethanalkylierungen mit Dimethylcarbonat

| Beispiel | Ausgangsprodukt | Katalysator | Hauptprodukt | Ausbeute | Tage | Bemerkungen |
|---|---|---|---|---|---|---|
| 4 | 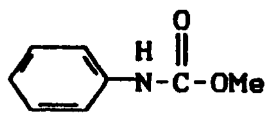 | TBAB | 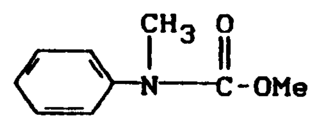 | 93% | 1 | Tetramethylensulfon |
| 5 | | TMBAC | | 78% | 14 | - |
| 6 | 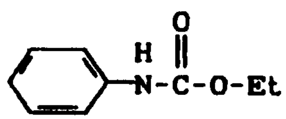 | TMBAC | 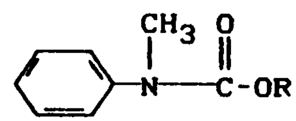 | 82% | 14 | R=$CH_3$, Et 45:55 |
| 7 | 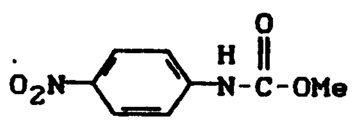 | TBAB | 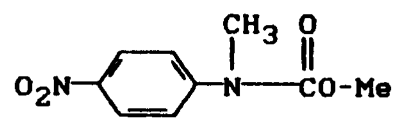 | 54% | 1 | - |

Urethanalkylierungen mit Dimethylcarbonat

| Beispiel | Ausgangsprodukt | Katalysator | Hauptprodukt | % Ausbeute | Tage | Bemerkungen |
|---|---|---|---|---|---|---|
| 8 | Toluylendiisocyanat-bis-methyl-urethan | TMBAC | 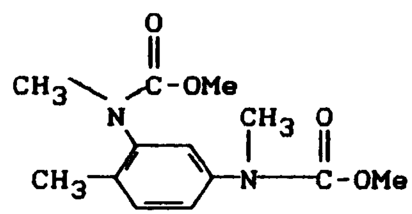 | 68 | 11 | neben Monoalkyl |
| 9 | | TBAB | | 90 | 5 | - |
| 10 | 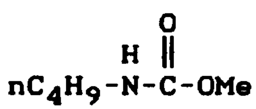 | TMBAC | 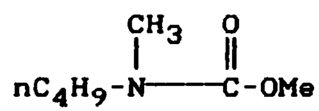 | 39 | 11 | Ausg. Produkt Tetramethylsulfon |
| 11 | Hexamethylendiisocyanat-bis-methylurethan | TBAB | 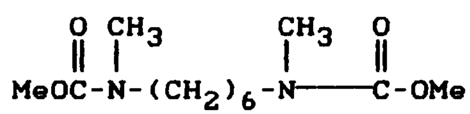 | 10 | 5 | Monoalkyl |
| 12 | | TBAB | | 74 | 18 | DMSO " |
| 13 | | TBAJ | | 63 | 14 | N-Methyl-pyrrolidon |
| 14 | | CTMAB | | 60 | 14 | " |

Beispiel 15

Bis-N-Methyl-2,4-TDI-Bismethylurethan

Zu 23,8 g (0,1 mol) 2,4-TDI-Bismethylurethan in 200 ml Dimethylcarbonat gibt man bei Raumtemperatur 10 g (0,072 mol) getrocknetes und gemahlenes Kaliumcarbonat und 2 g (0,01 mol) Trimethylbenzylammoniumchlorid. Nach 15 Tagen Rühren bei Rückfluß (94 - 97°C) ist kein Ausgangsmaterial mehr vorhanden. Es wird abgekühlt, der unlösliche Anteil abfiltriert und die Mutterlauge einrotiert. Das erhaltene Öl wird am Kugelrohrofen bei 200°C/0,01 mbar destilliert. Man erhält 14,7 g des gewünschten Endproduktes (80% d. Th.) neben 10% des monobenzylierten Produktes der Formel

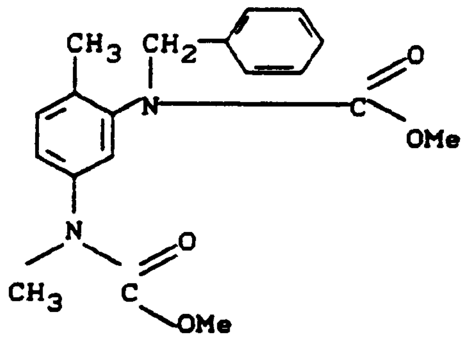

Beispiel 16-25

Die folgende Tabelle stellt zusammenfassend erfindungsgemäß durchgeführte Reaktionen unter Verwendung anderer Dialkylcarbonate vor. Aus dieser Gruppe ist Diethylcarbonat besonders geeignet.

**Tabelle 2**: Alkylierung von Urethanen (Kat.: TBAB)

| Beispiel | Ausgangsprodukt | Reagens | Hauptprodukt | Tage | Ausbeute | Cosolv./ Nebenp. |
|---|---|---|---|---|---|---|
| 16 | 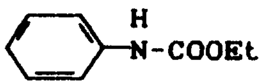 | DEC | 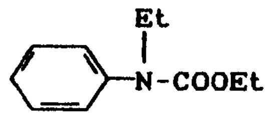 | 5 | 51% | - |
| 17 | | | | 3 | 83% | DMSO |
| 18 | | | | 2 | 81% | NMP |
| 19 | 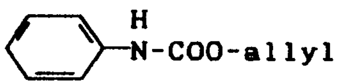 | ALLC | 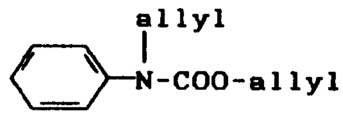 | 3 | 20% | - |
| 20 | | | | 4 | 35% | DMSO |
| 21 | 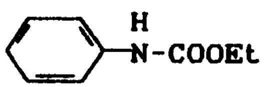 | ALLC | 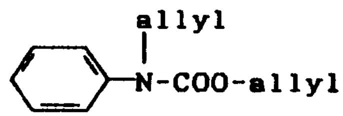 | 7 | 44% | 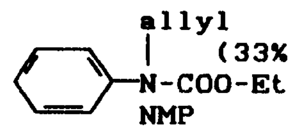 (33%) |
| 22 | | | | 3 | 87% | NMP |
| 23 | 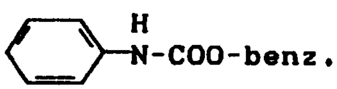 | BENC | 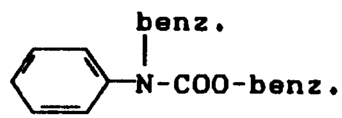 | 4 | 81% | - |
| 24 | | | | 2 | 20% | DMSO |
| 25 | | | | 4 | 89% | NMP |

DEC = Diethylcarbonat
ALLC = Diallylcarbonat
Benc = Dibenzylcarbonat

Beispiel 26 bis 31

Die folgenden Beispiele zeigen die Vielfalt der für das erfindungsgemäße Verfahren geeigneten Alkali/Erdalkalicarbonate.

Grundansatz:

| | |
|---|---|
| 10 g | N-Phenyl-o-methylurethan |
| 150 g | Dimethylcarbonat |
| 1 g | Tetrabutylammoniumbromid |
| 5 g | Base / Anführung wie Beispiel 1. |

**Ausbeuten an N-Phenyl-N-Methyl-o-methylurethan in %**

| Beispiel | 26 | 27 | 28 | 29 | 30 | 31 |
|---|---|---|---|---|---|---|
| Base | $Na_2CO_3$ | $K_2CO_3$ | $K_2CO_3$. Pyridin 1:1 | $CaCO_3$ | $MgCO_3$ | $Mgo \cdot MgCO_3$ |
| Ausbeute (2d/95° C) | 99,0 | 96,5 | 98,2 | 99,5 | 98,6 | 68,2 |
| Ausbeute (3d/95° C | * | * | 99,3 | * | * | 88,8 |

*) Reaktion n. 2d abgebrochen

Beispiel 32

a. Herstellung eines OH-endständigen Oligourethans

Zu 208 g (2,0 Mole) Neopentylglykol in 203 g trockenem Dimethylsulfoxid tropft man unter Rühren bei 50-70°C 261 g (1,5 Mol) 2,4-Tolylendiisocyanat und rührt nach Steigerung der Temperatur auf 80°C ca. 2 Stunden bis sich IR-spektroskopisch keine Isocyanatgruppen mehr nachweisen lassen. Man erhält eine 70 %ige Lösung eines OH-endständigen Oligourethans in Dimethylsulfoxid. Das rechnerische Molekulargewicht beträgt 940.

b. Erfindungsgemäßes Verfahren zur Herstellung des homologen N-Methylproduktes

Obiger Absatz wird mit 700 ml Dimethylcarbonat, 3 g Tetrabutylammoniumbromid und 25 g Kaliumcarbonat versetzt und 4 Tage bei einer Innentemperatur von 95°C gerührt. Nach Abkühlen, Absaugen und Entfernen der Lösemittel erhält man ein viskoses, schwachgelbes, nicht kristallisierendes Harz in einer Ausbeute von 99 %.

Eine Probe dieses Materials wird zur Entfernung von Katalysatorresten zwischen Wasser und Methylenchlorid verteilt, die Methylenchloridphase mit Wasser gewaschen, getrocknet und vom Methylenchlorid befreit. Der Alkylierungsgrad wird mit Hilfe der Protonenresonanz bestimmt. Er betrug 93,6 %.

Beispiel 33

Herstellung eines OH-endständigen Oligourethans

Zu einer Lösung aus 146 g (1,237 m) Hexandiol (2,5) in 200 ml Dimethylcarbonat tropft man bei 60°C 145 g (0,833 m) 2,4-Tolylendiisocyanat und rührt bei 90°C solange, bis IR-spektroskopisch kein Isocyanat mehr nachweisbar ist. Man erhält quantitativ eine 58,45 %ige Lösung eines OH-endständigen Oligourethans in Dimethylcarbonat vom berechneten Molekulargewicht 1745. Es handelt sich um ein kristallisierendes Harz.

Herstellung des Homologen N-Methylproduktes

Man verdünnt obige Lösung mit weiteren 300 ml Dimethylcarbonat und setzt 30 g Kaliumcarbonat und 3 g Tetrabutylammonium-bromid zu. Der Ansatz wird 9 Tage bei 95°C gerührt. Nun wird abgekühlt, das Kaliumcarbonat abfiltriert, die organische Lösung zweimal mit je 150 ml Wasser gewaschen, das Lösungsmittel abdestilliert und vom Endprodukt mit Hilfe der Protonenresonanz der Alkylierungsgrad bestimmt. Er beträgt 97,3 % der Theorie. Man erhält ein viskoses, nicht kristallisierendes hellgefärbtes Harz in einer Ausbeute von 95 % der Theorie.

## Patentansprüche

1. Verfahren zur Herstellung N,N-disubstituierter Mono- und Oligourethane, dadurch gekennzeichnet daß N-substiutierte Mono- und Oligourethane mit Dialkylcarbonaten der Formel

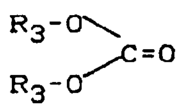

zum Einsatz,
in der

$R^3$ einen gegebenenfalls ungesättigten, gegebenenfalls verzweigten aliphatischen oder araliphatischen Kohlenwasserstoffrest mit 1-18, bevorzugt 1-10 C-Atomen, der weitere funktionelle Gruppen tragen kann,

bedeutet,

in Gegenwart von mindestens äquivalenten Mengen an Alkalicarbonaten und/oder Erdalkalicarbonaten und in Gegenwart von 1-10 Mol-% - bezogen auf die Urethangruppen - eines Phasentransferkatalysators in Gegenwart von überschüssigem Dialkylcarbonat und/oder eines aprotischen Lösungsmittels umgesetzt werden.

2. Verfahren nach Anspruch 1, dadurch gekennzeichnet, daß als Dialkylcarbonat Dimethylcarbonat verwendet wird.

3. Verfahren nach einem der Ansprüche 1 oder 2, dadurch gekennzeichnet, das Kaliumcarbonat in trockener, gepulverter Form als Alkalicarbonat verwendet wird.

4. Verfahren nach einem der Ansprüche 1 bis 3, dadurch gekennzeichnet, daß die Umsetzung ohne Lösungsmittels in überschüssigem Dialkylcarbonat durchgeführt wird.

5. Verfahren nach einem der Ansprüche 1 bis 3, dadurch gekennzeichnet, daß die Umsetzung in Di methylsulfoxid, Dimethylformamid, N-Methylpyrrolidon oder Tetramethylensulfon durchgeführt wird.

6. Verfahren nach einem der Ansprüche 1 bis 5, dadurch gekennzeichnet, daß als Phasentransferkatalysatoren Tetraalkylammoniumchloride, -bromide oder -jodide verwendet werden.

## Claims

1. A process for the production of N,N-disubstituted mono- and oligourethanes, characterized in that N-substituted mono- and oligourethanes are reacted with dialkyl carbonates corresponding to the formula:

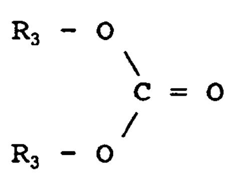

in which

$R^3$ is an optionally unsaturated, optionally branched aliphatic or araliphatic $C_{1-18}$ and preferably $C_{1-10}$ hydrocarbon radical which may bear other functional groups,

in the presence of at least equivalent quantities of alkali metal carbonates and/or alkaline earth metal carbonates and in the presence of 1 to 10 mol-%, based on the urethane groups, of a phase transfer catalyst in the presence of excess dialkyl carbonate and/or an aprotic solvent.

2. A process as claimed in claim 1, characterized in that dimethyl carbonate is used as the dialkyl carbonate.

3. A process as claimed in claim 1 or 2, characterized in that potassium carbonate in dry, powdered form is used as the alkali metal carbonate.

4. A process as claimed in any of claims 1 to 3, characterized in that the reaction is carried out in the absence of a solvent in excess dialkyl carbonate.

5. A process as claimed in any of claims 1 to 3, characterized in that the reaction is carried out in dimethyl sulfoxide, dimethyl formamide, N-methyl pyrrolidone or tetramethylene sulfone.

6. A process as claimed in any of claims 1 to 5, characterized in that tetraalkyl ammonium chlorides, bromides or iodides are used as the phase transfer catalysts.

## Revendications

1. Procédé de production de mono- et oligouréthannes portant deux substituants sur l'azote, caractérisé en ce qu'on utilise des mono- et oligo-uréthannes substitués sur l'azote, avec des carbonates de dialkyle de formule

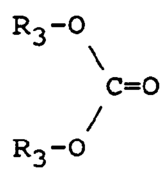

dans laquelle

$R^3$ est un reste d'hydrocarbure aliphatique ou araliphatique éventuellement non saturé et le cas échéant ramifié ayant 1 à 18, de préférence 1 à 10 atomes de carbone, qui peut porter d'autres groupes fonctionnels,

en présence de quantités au moins équivalentes de carbonates alcalins et/ou de carbonates alcalino-terreux et en présence de 1 à 10 moles % - par rapport aux groupes uréthanne - d'un catalyseur de transfert de phase en présence de carbonate de dialkyle en excès et/ou d'un solvant aprotique.

2. Procédé suivant la revendication 1, caractérisé en ce qu'on utilise le carbonate de diméthyle comme carbonate de dialkyle.

3. Procédé suivant l'une des revendications 1 et 2, caractérisé en ce qu'on utilise comme carbonate alcalin le carbonate de potassium sous la forme pulvérisée sèche.

4. Procédé suivant l'une des revendications 1 à 3, caractérisé en ce qu'on conduit la réaction en l'absence d'un solvant dans du carbonate de dialkyle en excès.

5. Procédé suivant l'une des revendications 1 à 3, caractérisé en ce que la réaction est conduite dans du diméthylsulfoxyde, du diméthylformamide, de la N-méthylpyrrolidone ou de la tétraméthylènesulfone.

6. Procédé suivant l'une des revendications 1 à 5, caractérisé en ce qu'on utilise comme catalyseurs de transfert de phase des chlorures, bromures ou iodures de tétraalkylammonium.